(19) Europäisches Patentamt
European Patent Office
Office européen des brevets

(11) Publication number: **0 450 705 A1**

# (12) EUROPEAN PATENT APPLICATION

(21) Application number: **91200703.6**

(22) Date of filing: **27.03.91**

(51) Int. Cl.5: **C07D 281/08**, C07D 281/10, A61K 31/55

(30) Priority: **31.03.90 NL 9000763**

(43) Date of publication of application: **09.10.91 Bulletin 91/41**

(84) Designated Contracting States: **BE CH DE FR GB LI NL**

(71) Applicant: **STAMICARBON B.V.**
**Mijnweg 1**
**NL-6167 AC Geleen(NL)**

(72) Inventor: **Hulshof, Lumbertus Albregt**
**Bosbeeklaan 1**
**NL-5991 MA Baarlo(NL)**
Inventor: **Kuilman, Thijs**
**Gaspeldoornstraat 44**
**NL-5925 BA Venlo-Blerick(NL)**

## (54) Process for preparing 1,5-benzothiazepin derivatives.

(57) The invention relates to a process for preparing a preferably stereoisomerically pure 1,5-benzothiazepin derivative with the general formula (I) by the cyclization of an ester of the corresponding 2-hydroxy-3-(4-$R_3$-phenyl)--3-(2-aminoarylthio)propanoic acid with the general formula (II) in the presence of a base and in an aprotic, polar solvent

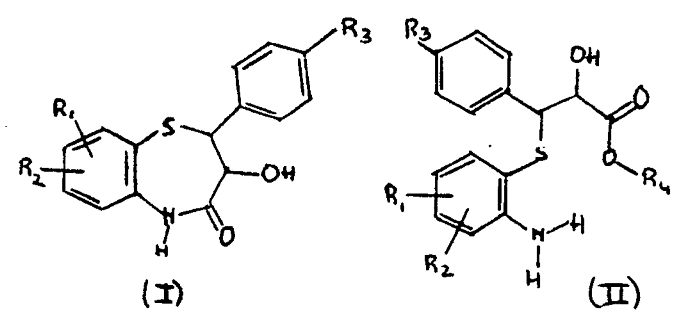

(I) (II)

where $R_1$ and $R_2$, each independently, represent hydrogen, halogen, or an alkyl group with 1-6 carbon atoms or together with the phenyl group to which they are attached from a naphtalene group, $R_4$ represents a residual group with 1-20 carbon atoms and $R_3$ a hydrogen atom, a hydroxy group or an alkoxy group with 1-6 carbon atoms, at which a 2-hydroxy-3-(4-$R_3$-phenyl)-3-(2-aminoarylthio)propanoic acid ester with the general formula (II) is cyclized in the presence of an alkali metal alkanolate as base.

The invention also relates to a process for the preparation of alkylated and/or acylated 1,5-benzothiazepin derivatives and to the new compounds of (2X,3Y)-2-phenyl-3-hydroxy-2,3-dihydro-1,5-benzothiazepin-4(5H)-one, (2X,3Y)-2-phenyl-3-hydroxy-5-[2-(dimethylamino)ethyl]-2,3-di hydro-1,5-benzothiazepin-4(5H)-one and (2X,3Y)-2-phenyl--3-acetyloxy-5-[2-(dimethylamino)ethyl]-2,3-dihydro-1,5-benz othiazepin-4(5H)-one, where X and Y each independently represent the R or S configuration.

Application of 1,5-benzothiazepin derivatives, obtained according to the process of the present invention, in the preparation of pharmaceuticals and particularly in the preparation of diltiazem.

EP 0 450 705 A1

The invention relates to a process for preparing a 1,5-benzothiazepin derivative with the general formula (I) by the cyclization of an ester of the corresponding 2-hydroxy-3-(4-$R_3$-phenyl)-3-(2-aminoarylthio)-propanoic acid with the general formula (II) in the presence of a base and in an aprotic, polar solvent,

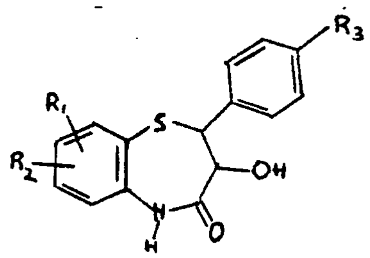

(I)

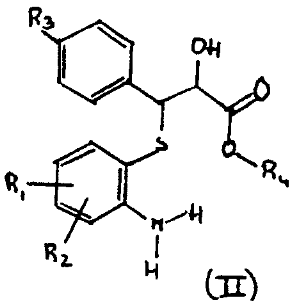

(II)

where $R_1$ and $R_2$, each independently, represent hydrogen, halogen, or an alkyl group with 1-6 carbon atoms or together with the phenyl group to which they are attached form a naphtalene group, $R_4$ represents a residual group with 1-20 carbon atoms and $R_3$ a hydrogen atom, a hydroxy group or an alkoxy group with 1-6 carbon atoms.

Such a process is known from JP-A-60-78973. In that patent publication a process is described for the preparation of cis-1,5-benzothiazepin derivatives by the cyclization of a methyl ester of the corresponding 2-hydroxy-3-(4-methoxyphenyl)-3-(2-aminophenylthio)propanoic acid in the presence of metal hydrides, such as sodium hydride, as a base, and an aprotic, polar solvent, such as dimethylsulphoxide. To this reaction mixture is added also, after some time, an alkylating agent, particularly dimethylaminoethyl chloride. Owing to this, an alkylation process takes place also, in addition to the cyclization, so that the cis-2-(4-methoxyphenyl)-3-hydroxy-5-[2-(dimethylamino)ethyl]-2,3-dihydro-1,5-benzothiazepin-4(5H)-one is obtained directly.

A disadvantage of the said process is that highly reactive metal hydrides are used, resulting in reaction mixtures hazardous for industrial applications. Moreover, it has been found that, when metal hydrides are used, the efficiency is low.

The object of the invention, therefore, is to provide a simple process avoiding the said disadvantages.

This object is achieved according to the process of the present invention in that a 2-hydroxy-3-(4-$R_3$-phenyl)-3-(2-aminoarylthio)propanoic acid ester with the general formula (II) is cyclized in the presence of an alkali metal alkanolate as base.

The result will be that the cyclization is effected in a simple manner using mild reagents, which is attractive for industrial application. Moreover, a higher efficiency is reached if, instead of metal hydrides, alkali metal alkanolates are used.

The choice of $R_4$ in the general formula (II) is not critical.

The usual residual ester groups will qualify, such as alkyl groups with 1-6 carbon atoms.

If the object is the preparation of stereoisomerically pure products, preference is given in the process according to the present invention to the use of stereoisomerically pure starting products.

A stereoisomerically pure starting product is in this connection understood to mean a starting product wholly or largely consisting of one stereoisomer such as, for instance, a (2X,3Y)-2-hydroxy-3-(4-$R_3$-phenyl)-3-(2-aminoarylthio)propanoic acid ester, where X and Y each independently represent the R- or S-configuration.

The advantage is then that no mixture of stereoisomers is obtained such as, for instance, (2S,3S)-and (2R,3R)-1,5-benzothiazepin derivatives, in case a threo-2-hydroxy-3-(4-$R_3$-phenyl)-3-(2-aminoarylthio)-propanoic acid ester is used.

Moreover, when stereoisomerically pure starting products are used, there will be a smaller loss of raw materials, because the stereoisomeric starting product with the undesired configuration need not be converted and no undesired stereoisomers are produced as waste products either. Besides, in order to obtain a stereoisomerically pure product from a mixture of stereoisomers, an additional resolution step is required in which generally the desired stereoisomerically pure product can only with difficulty be recovered in an optically pure state.

The (2X,3Y)-2-hydroxy-3-(4-$R_3$-phenyl)-3-(2-aminoarylthio)-propanoic acid alkyl ester is preferably a (2S,3S)-2-hydroxy-3-(4-methoxyphenyl)-3-(2-aminophenylthio)-propanoic acid alkyl ester and particularly the methyl ester thereof.

As a result, the cyclization product obtained is (+)-(2S,3S)-2-(4-methoxyphenyl)-3-hydroxy-2,3-dihydro-1,5-benzothiazepin-4(5H)-one, which is particularly desired as an intermediate in the preparation of diltiazem.

Diltiazem is the name generally used for (+)-(2S,3S)-2-(4-methoxyphenyl)-3-acetyloxy-5-[2-(dimethylamino)ethyl]-2,3-dihydro-1,5-benzothiazepin-4(5H)-one and is known from, for instance, US-A-3,562,257.

Diltiazem is a calcium antagonist used in the event of cardiovascular disorders, such as angina pectoris. Besides, it is used also against disturbances of the rhythm of the heart and against hypertension.

Other suitable (2X,3Y)-2-hydroxy-3-(4-$R_3$-phenyl)-3-(2-aminoarylthio)propanoic acid alkyl esters are, inter alia, the methyl, ethyl, propyl, butyl, isobutyl, t-butyl, pentyl or hexyl esters of, for instance, (2X,3Y)-2-hydroxy-3-phenyl-3-(2-aminophenylthio)propanoic acid, (2X,3Y)-2-hydroxy-3-phenyl-3-(2-amino-5-chlorophenylthio)pro panoic acid (2X,3Y)-2-hydroxy-3-(4-hydroxyphenyl)-3-(2-aminophenylthio)propanoic acid, (2X,3Y)-2-hydroxy-3-(4-methoxyphenyl)-3-(2-aminophenylthio)propanoic acid, (2X, 3Y)-2-hydroxy-3-(4-ethoxyphenyl)-3-(2-aminophenylthio)-propanoic acid, (2X,3Y)-2-hydroxy-3-(4-propoxyphenyl)-3-(2-aminophenylthio)propanoic acid, (2X,3Y)-3-[(2-amino-1-naphthalenyl)thio]-2-hydroxy-3-(4-methoxyphenyl)-propanoic acid.

From these esters the corresponding 1,5-benzothiazepin derivatives can be prepared, from which optionally diltiazem or substances analogous to diltiazem can be prepared.

The above-mentioned esters can be obtained by linking an aminoarylthiol to an ester of the corresponding phenylglycidic acid, as described in, for instance, JP-A-61-145159.

The base applied is an alkali metal alkanolate, preferably chosen from the group of alkali metal alkanolates with 1-6 carbon atoms. The base can be added to the reaction mixture in a solid form, as well as dissolved in the corresponding alcohol. Examples of such bases are, inter alia, sodium methoxide, potassium methoxide, lithium methoxide, sodium ethoxide, potassium ethoxide, sodium propoxide, potassium isopropoxide, potassium tertiary butoxide or mixtures thereof. Most preference is given to sodium methoxide or potassium tertiary butoxide.

Suitable aprotic polar solvents are, for instance, dimethyl sulphoxide, N,N-dimethylformamide, sulpholane, acetonitrile, N,N-dimethylacetamide, N,N'-dimethylimidazolidinone or N-methylpyrrolidone. The preferred solvent is N,N-dimethylformamide or N,N'-dimethylimidazolidinone.

The cyclization usually takes place at a temperature of -20 °C to +50 °C and preferably of -10 °C to +15 °C.

In a suitable embodiment the cyclization takes place in an inert atmosphere, for instance nitrogen. The (2X,3Y)-2-hydroxy-3-(4-$R_3$-phenyl)-3-(2-aminoarylthio) propanoic acid ester is then dissolved in an aprotic polar solvent, for instance N,N-dimethylformamide, and cooled during stirring to, for instance, 0 °C. Subsequently, an alkali metal alkanolate, for instance sodium methoxide, is added, whether or not dissolved in methanol, upon which cyclization takes place to form the corresponding 1,5-benzothiazepin derivative by continuing the stirring process for some time at this temperature. The reaction mixture is poured onto iced water. The suspension is subsequently isolated at a low temperature, for instance by filtration or centrifugation at 0 °C, and the resulting solid is washed with water at room temperature. Thus a (2X,3Y)-1,5-benzothiazepin derivative is rapidly obtained in a high yield in a relatively simple manner.

The invention also relates to the preparation of alkylated and/or acylated 1,5-benzothiazepin derivatives with the general formula (III),

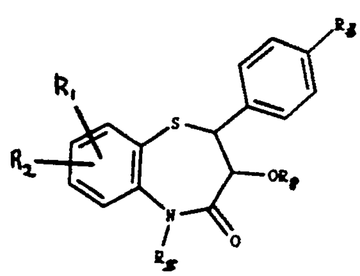

(III)

where $R_5$ represents a hydrogen atom or an $R_6R_7N(CH_2)n$-group, where n equals 1, 2, 3 or 4, $R_6$ and $R_7$ are each independently an alkyl, aryl, alkaryl or aralkyl group with 1-10 carbon atoms, and where $R_8$ represents a hydrogen atom or an $R_9(CO)$-group, where $R_9$ is an alkyl, aryl, alkaryl or aralkyl group with 1-10 carbon atoms, and to the use of the resulting 1,5-benzothiazepin derivatives in the preparation of pharmaceuticals and more particularly in the preparation of diltiazem or substances analogous to diltiazem.

Here again preference should be given to the use of stereoisomerically pure starting products in order to obtain (2X-3Y)-1,5-benzothiazepin derivatives with the general formula (III). Examples of such derivatives are (2S,3S)-2-(4-methoxyphenyl)-3-hydroxy-5-[2(dimethylamino)-ethyl]-9-choro-2,3-dihydro-1,5-benzothiazepin-4(5H)-one, (2R,3R)-2-(4-methoxyphenyl)-3-acetyloxy-2,3-dihydro-1,5-benzothiazepin-4(5H)-one, (2R,3S)-2-(4-phenyl)-3-hydroxy-5-[2-(diethylamino)propyl]-2,3-dihydro-1,5-benzothiazepin-4-(5H)-one, (2S,3S)-2,3-dihydro-3-hydroxy-2(4-methoxyphenyl)-naphta-[1,2-b]-1,4-thiazepin-4 (5H)-one.

It is a further advantage of the process according to the present invention that the resulting cyclization product, more particularly (+)-(2S,3S)-2-(4-methoxyphenyl)-3-hydroxy-2,3-dihydro-1,5-benzothiazepin-4-(5H)-one, can be subjected direct to an alkylation process without first being isolated, in which alkylation process the alkylated product is obtained in a high yield. This alkylated product can optionally, again without intermediate recovery, be converted direct via an acylation process into the desired alkylated and acylated 1,5-benzothiazepin derivative. If so desired, the cyclization product obtained can also be subjected to an acylation reaction direct.

For the benefit of the alkylation and/or acylation reaction after the cyclization an alkali metal bicarbonate is mostly added initially to the reaction mixture, and that in such an amount that in the alkylation and/or acylation hardly any alkali metal alkanolate is left. The alkali metal bicarbonate used is preferably sodium bicarbonate or potassium bicarbonate, or a mixture thereof.

The cyclization product is alkylated, for instance, by the addition of 1-2 equivalents of an $R_6R_7N(CH_2)_n$-halide, optionally as HCl salt, where $R_6$, $R_7$ and n are defined as stated above.

The halide used in the preparation of diltiazem is mostly dimethylaminoethyl chloride or the HCl salt thereof. The alkylated cyclization product can subsequently, without further recovery, be acylated, whether or not after replacement of the solvent.

The acylation of the alkylated or non-alkylated cyclization product usually takes place in the presence of a suitable acylating agent and a basic catalyst such as, for instance, dimethylaminopyridine.

The acylating agent used may be an acid chloride with the general formula $R_9COCl$ or an acid anhydride with the general formula $(R_9CO)_2O$, where $R_9$ preferably represents an alkyl, aryl, alkaryl or aralkyl group with 1-10 carbon atoms.

Suitable acid chlorides or anhydrides are acetyl chloride, propanoyl chloride, butanoyl chloride, isobutanoyl chloride, pentanoyl chloride, benzoyl chloride, acetic anhydride, propanoic anhydride, butanoic anhydride, pentanoic anhydride or benzoic anhydride. The acid anhydride used is preferably acetic anhydride.

The resulting reaction product can conveniently be further processed by first evaporating the solvent, subsequently taking up the residue in an alcohol, for instance isopropanol, and introducing HCl gas into it. Thus alkylated and/or acylated 1,5-benzothiazepin derivatives are obtained as the HCl salt.

The invention also relates to the new compounds of (2X,3Y)-2-phenyl-3-hydroxy-2,3-dihydro-1,5-benzothiazepin-4( 5H)-one, (2X,3Y)-2-phenyl-3-hydroxy-5-[2-(dimethylamino)-ethyl]-2,3-dihydro-1,5-benzothiazepin-4(5H)-one and (2X,3Y)-2-phenyl-3-acetyloxy-5-[2-(dimethylamino)ethyl]-2,3-dihydro-1,5-benzothiazepin-4(5H)-one, which can be obtained in an optically pure form and in a high yield by applying the process according to the invention. Here the X and Y each separately represent the R or S configuration. The invention therefore also relates to these 1,5-benzothiazepin derivatives.

The invention will be further elucidated by the following examples without, however, being limited by them.

Example I

In a four-necked 5-litre flask with stirrer and thermometer a solution of 500 grammes (+)-(2S,3S)-2-hydroxy-3-(4-methoxyphenyl)-3-(2-aminophenylthio)propanoic acid methyl ester in 3.75 litres N,N-dimethylformamide was cooled to 0°C in a nitrogen atmosphere. Subsequently, 800 ml of a 30% solution of sodium methoxide in methanol was added gradually in one hour, in which addition the temperature was kept at 0°C. The reaction mixture was stirred at this temperature for 1.5 hours more, upon which it was poured out on 5 litres iced water. The resulting suspension was stirred for one hour more and subsequently centrifuged at 0°C. At room temperature, the resulting solid was washed with water and dried. 401.4 grammes (88.8%) (+)-(2S,3S)-2-(4-methoxyphenyl)-3-hydroxy-2,3-dihydro-1,5-benzothiazepin-4(5H)- one was obtained. $[\alpha]^{20}_D = +114.0°$ (c = 0.4 ; MeOH).

Example II

Example I was repeated, but this time the solution was cooled to -5°C and, instead of sodium

methoxide, 212,5 grammes potassium tertiary butoxide was added at this temperature at once. In the process the temperature was allowed to rise to 0 °C and the reaction mixture was stirred for half an hour at this temperature, upon which it was poured out on iced water and subsequently received further treatment as indicated in example I. 423.8 grammes (93.9%) (+)-(2S,3S)-2-(4-methoxyphenyl)-3-hydroxy-2,3-dihydro-1,5-be nzothiazepin-4(5H)-one was obtained.
$[\alpha]^{20}_D = +114.9°$ (c = 0.4 ; MeOH).

Example III

Example I was repeated, but this time 162.5 grammes sodium methoxide was added as a solid at once. The reaction mixture was stirred for one hour at 0 °C, upon which it was poured out on iced water and subsequently received further treatment as indicated in example I. 412.5 grammes (91.4%) (+)-(2S,3S)-2-(4-methoxyphenyl)-3-hydroxy-2,3-dihydro-1,5-benzothiazepin-4(5H)-one was obtained.
$[\alpha]^{20}_D = +115.1°$ (c = 0.4 ; MeOH).

Example IV

Example III was repeated, but this time the ester was dissolved in 3.75 litres N,N'-dimethylimidazolidinone, upon which the reaction mixture was cooled to -5 °C and subsequently stirred at this temperature. 378.8 grammes (83.8%) (+)-(2S,3S)-2-(4-methoxyphenyl)-3hydroxy-2,3-dihydro-1,5-benzothiazepin-4(5H)-one was obtained.
$[\alpha]^{20}_D = +114.1°$ (c = 0.4 ; MeOH).

Comparative experiment

In a four-necked 5-litre flask with stirrer and thermometer a solution of 500 grammes (+)-(2S,3S)-2-hydroxy-3-(4-methoxyphenyl)-3-(2-aminophenylthio)propanoic acid methyl ester in 3.75 litres N,N-dimethylformamide was cooled to -5 °C in a nitrogen atmosphere. Subsequently, 55 grammes (80% wt) sodium hydride in paraffin oil was added to this solution, at which the temperature was kept constant. The reaction mixture was stirred at this temperature for 1.5 hours. After that a further 12.5 grammes (80% wt) sodium hydride in paraffin oil was added to the reaction mixture at this temperature in order to allow the reaction to be completed. After stirring for one hour more, the reaction mixture was poured out on 5 litres iced water. The resulting suspension was stirred for one hour and subsequently filtered at 0 °C. At room temperature, the solid obtained was washed with water and dried. 333.8 grammes (73.9%) (+)-(2S,3S)-2-(4-methoxyphenyl)-3-hydroxy-2,3-dihydro-1,5-be nzothiazepin-4(5H)-one was obtained.
$[\alpha]^{20}_D = +112.9°$ (c = 0.4 ; MeOH).

Example V

In a four-necked 10-litre flask with stirrer and thermometer a solution of 500 grammes (+)-(2S,3S)-2-hydroxy-3-(4-methoxyphenyl)-3-(2-aminophenylthio)propanoic acid methyl ester in 3.75 litres N,N-dimethylformamide was cooled to -15 °C in a nitrogen atmosphere. Subsequently, 788 ml of a 30% solution of sodium methoxide in methanol was added gradually in 1 hour, at which the temperature was kept at -15 °C. The reaction mixture was stirred at this temperature for one hour more, upon which the temperature was made to rise to 0 °C in one hour. Subsequently, 162.5 grammes sodium bicarbonate and 287.5 grammes dimethylaminoethyl chloride-hydrochloride were added. The reaction mixture was heated to 90 °C, upon which it was stirred at this temperature for one hour. After that, 1.25 litres solvent was evaporated under reduced pressure. Subsequently, at a temperature of 90 °C, 187.5 ml acetic anhydride and 12.5 grammes dimethylaminopyrridine were added. The reaction mixture was stirred for two hours and after that a further amount of 500 ml acetic anhydride was added in two hours. Subsequently, under reduced pressure, 2.5 litres solvent was evaporated, upon which 3.75 litres toluene and 3.75 litres water were added. Using sodium bicarbonate the pH was brought to 6 and the water phase was separated off. The organic phase was washed once again with 2.5 litres water. The organic phase was subsequently dried on MgSO4. After that 3.75 litres propanol was added and HCl gas was introduced to obtain a pH swing. The suspension thus obtained was filtered and washed with isopropanol. 425 grammes (63%) (+)-(2S,3S)-2-(4-methoxyphenyl)-3-acetyloxy-5-[2-( dimethylamino)ethyl]-2,3-dihydro-1,5-benzothiazepin-4( 5H)-one was obtained. The melting point was 210.2-211.7 °C and $[\alpha]^{20}_D = +116.0°$ (c = 1 ; $H_2O$).

Example VI

Example V was repeated up to and including the step in which the reaction mixture was stirred for 1 hour at 90° C. After that 3.7 litres solvent was evaporated under reduced pressure, upon which 2.5 litres toluene and 2.5 litres water were added. The water phase was separated off and the organic phase was heated up to reflux temperature (110° C), at which the remaining water was distilled off using a Dean-Stark assembly. Subsequently, 187.5 ml acetic anhydride and 3.75 grammes dimethylaminopyrridine were added. The reaction mixture was stirred for 2 hours at reflux temperature and subsequently cooled to room temperature, upon which 2.5 litres isopropanol was added. The reaction mixture was subsequently further cooled to -5° C and HCl gas was introduced to obtain a pH swing. The suspension thus obtained was filtered and washed with isopropanol. 425 grammes (63%) (+)-(2S,3S)-2-(4-methoxyphenyl)-3-acetyloxy-5-[2-(dimethylamino)ethyl]-2,3-dihydro-1,5-benzothiazepin-(5H)-one was obtained. The melting point was 207.0-210.7° C and $[\alpha]^{20}_D$ = +111.0° (c = 1 ; $H_2O$).

Examples I-IV and the comparative experiment clearly show that the cyclization product can be obtained in a high yield and with a high purity using mild reagents. Moreover, examples V and VI show that, if so desired, the cyclization product can be alkylated and acylated with a good yield and a good purity without an intermediate recovery of the reaction product.

Example VII

(+)-(2S,3S)-8-Chloro-3-hydroxy-2-(4-methoxyphenyl) -2,3-dihydro-5H-1,5-benzothiazepin-4-one

In a three-necked 100-ml flask with stirrer and thermometer a solution of 6 grammes (+)-(2S,3S)-2-hydroxy-3-(4-methoxyphenyl)-3-(2-amino-5-chlorophenylthio)propanoic acid methyl ester in 41 ml N,N-dimethylformamide was cooled to -5° C in a nitrogen atmosphere. Subsequently, at this temperature, 2.3 grammes potassium tertiary butoxide was added at once. The reaction mixture was stirred for one hour at -5 to 0° C, upon which it was poured out on 100 ml iced water. The resulting suspension was stirred for 1/2 hour more and subsequently filtered at 0° C. At room temperature, the solid obtained was washed with water and dried. 4.42 grammes (82.2%) (+)-(2S,3S)-8-chloro-3-hydroxy-2-(4-methoxyphenyl)-2,3-dihydro-5H-1,5-benzothiazepin-4-one was obtained.
$[\alpha]^{20}_D$ = +94.4° (c = 0.1 ; DMF); lit. (EP-A-0378455): +91.9° (c = 0.1 ; DMF).

Comparative experiment

In a three-necked 100-ml flask with stirrer and thermometer a solution of 6 grammes (+)-(2S,3S)-2-hydroxy--3-(4-methoxyphenyl)-3-(2-amino-5-chlorophenylthio)propanoic acid methyl ester in 41 ml N,N-dimethylformamide was cooled to -5° C in a nitrogen atmosphere. Subsequently, 0.63 gramme (80% wt) sodium hydride in paraffin oil was added to this solution, upon which the temperature was kept at -5 to 0° C. The reaction mixture was stirred at this temperature for 1.5 hours and subsequently poured out on 100 ml iced water. The suspension formed was stirred for 1/2 an hour and subsequently filtered at 0° C. At room temperature, the solid obtained was washed with water and dried. 3.81 grammes (70.8%) (+)-(2S,3S)-8-chloro-3-hydroxy-2-(4-methoxyphenyl)-2,3-dihydro-5H-1,5-benzothiazepin-4-one was obtained.
$[\alpha]^{20}_D$ = +100.1° (c = 0.1 ; DMF).

Example VIII

(+)-(2S,3S)-2,3-dihydro-3-hydroxy-2-(4-methoxyphenyl)-naphtho-[1,2-b]-4-thiazepin-4(5H)-one

In a three-necked 100-ml flask with stirrer and thermometer a solution of 10.5 grammes (+)-(2S,3S)-3-[-(2-amino-1-naphthalenyl)thio]-2-hydroxy-3-(4-methoxyphenyl)propanoic acid methyl ester in 50 ml N,N-dimethylformamide was cooled to -5° C in a nitrogen atmosphere. Subsequently, 3.9 grammes potassium tertiary butoxide was added to this solution, upon which the temperature was kept at -5 to 0° C. After stirring at this temperature for one hour a further amount of 1.5 grammes potassium tertiary butoxide and after two hours a further amount of 0.5 gramme potassium tertiary butoxide was added to complete the reaction. After stirring for 1/2 an hour more, the reaction mixture was poured out on 200 ml iced water. The resulting suspension was stirred for 1/2 an hour and subsequently filtered at 0° C. At room temperature, the solid obtained was washed with water and dried. 5.24 grammes (54.3%) (+)-(2S,3S)-2,3-dihydro-3-hydroxy-2-(4-methoxyphenyl)-naphtho-[1,2-b]-1,4-thiazepin-4(5H)-one was obtained. After recrystallization from acetonitrile, pure product was obtained with $[\alpha]^{20}_D$ = +24.9° (c = 0.5 in acetone); lit. (EP-A-343474) + 24.87 (c = 0.4 ; acetone).

Comparative experiment

In a three-necked 100-ml flask with stirrer and thermometer a solution of 10.5 grammes (+)-(2S,3S)-3-[-(2-aminonaphthalenyl)-thio]-2-hydroxy-3-(4-methoxyphenyl)-propanoic acid methyl ester in 50· ml N,N-dimethylformamide was cooled to -5° C in a nitrogen atmosphere. Subsequently, 1.2 grammes (80% wt) sodium hydride in paraffin oil was added to this solution, upon which the temperature was kept at -5 to 0° C. After stirring at this temperature for 1 1/2 hours a further amount of 42 grammes (80% wt) sodium hydride in paraffin oil was added to the reaction mixture in order to complete the reaction. After stirring for 1 1/2 hours more, the reaction mixture was poured out on 200 ml iced water. The resulting suspension was stirred for 1/2 an hour and subsequently filtered at 0° C. At room temperature, the solid obtained was washed and dried. 3.28 grammes (34%) (+)-(2S,3S)-2,3-dihydro-3-hydroxy-2-(4-methoxyphenyl)-naphtho-[1,2-b]-1,4-thiazepin-4(5H)-one was obtained as raw product.
$[\alpha]^{20}_D = +43.8^\circ$ (c = 0.5 ; acetone).

## Claims

1. Process for preparing a 1,5-benzothiazepin derivative with the general formula (I) by thecyclization of an ester of the corresponding 2-hydroxy-3-(4-$R_3$-phenyl)--3-(2-aminoarylthio)propanoic acid with the general formula (II) in the presence of a base and in an aprotic, polar solvent,

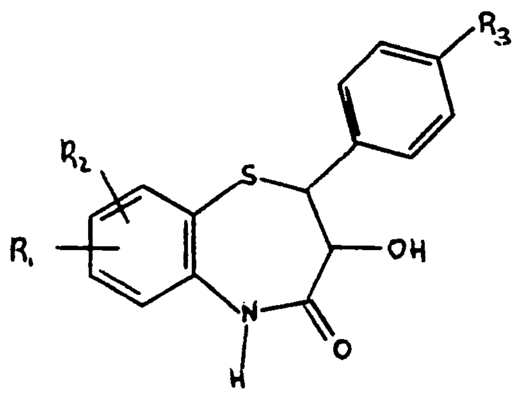

(I)

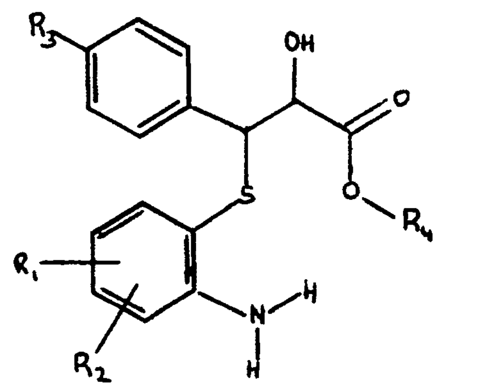

(II)

where where $R_1$ and $R_2$, each independently, represent hydrogen, halogen, or an alkyl group with 1-6 carbon atoms or together with the phenyl group to which they are attached from a naphtalene group, $R_4$ represents a residual group with 1-20 carbon atoms and $R_3$ a hydrogen atom, a hydroxy group or an alkoxy group with 1-6 carbon atoms, characterized in that a 2-hydroxy-3-(4-$R_3$-phenyl)-3-(2-aminoarylthio)propanoic acid ester with the general formula (II) is cyclized in the presence of an alkali metal alkanolate as base.

2. Process according to claim 1, characterized in that the ester used is a (+)-(2S,3S)-2-hydroxy-3-(4-methoxyphenyl)-3- (2-aminophenylthio)propanoic acid alkyl ester and more particularly the methyl ester thereof.

3. Process according to claim 1 or 2, characterized in that the alkali metal alkanolate is sodium methoxide or potassium tertiary butoxide.

4. Process for preparing an alkylated 1,5-benzothiazepin-derivative, characterized in that a 1,5-benzothiazepin derivative obtained according to any one of claims 1-3 is alkylated without intermediate recovery and after addition of an alkali metal bicarbonate, using an $R_6R_7N(CH2)n$-halide, where n equals 1, 2, 3 or 4, $R_6$ and $R_7$ are each independently an alkyl, aryl, alkaryl or aralkyl group with 1-10 carbon atoms.

5. Process for preparing an acylated 1,5-benzothiazepin derivative, characterized in that a 1,5-benzothiazepin derivative obtained according to any one of claims 1-4 is acylated without intermediate

recovery using a suitable acylating agent.

6. Process according to claim 5, characterized in that the acid anhydride is acetic anhydride.

7. Application of a (2X,3Y)-1,5-benzothiazepin derivative, obtained according to any one of claims 1-6, in the preparation of pharmaceuticals.

8. (2X,3Y)-2-phenyl-3-hydroxy-2,3-dihydro-1,5-benzothiazepin-4(5H)-one, with X and Y each independently representing the R or S configuration.

9. (2X,3Y)-2-phenyl-3-hydroxy-5-[2-(dimethylamino)ethyl]-2, 3-dihydro-1,5-benzothiazepin-4(5H)-one, with X and Y each independently representing the R or S configuration.

10. (2X,3Y)-2-phenyl-3-acetyloxy-5-[2-(dimethylamino)ethyl]-2,3-dihydro-1,5-benzothiazepin-4(5H)-one, with X and Y each independently representing the R or S configuration.

European Patent Office

# EUROPEAN SEARCH REPORT

Application Number

**EP 91 20 0703**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | PATENT ABSTRACTS OF JAPAN, vol. 9, no. 218 (C-301)[1941], 5th September 1985; & JP-A-60 78 973 (TOUSHIN CHEMICAL) 04-05-1985 | 1-7 | C 07 D 281/08<br>C 07 D 281/10<br>A 61 K 31/55 |
| Y | CHEMICAL ABSTRACTS, vol. 98, no. 11, 14th March 1983, page 564, abstract no. 89401a, Columbus, Ohio, US; & SU-A-956 474 (Z.F. SOLOMKO) 07-09-1982 | 1-7 | |
| Y | EP-A-0 081 234 (TANABE SEIYAKU)<br>* The whole document * | 1-10 | |
| D,Y | US-A-3 562 257 (H. KUGITA)<br>* The whole document * | 1-10 | |
| Y | US-A-3 646 008 (H. KUGITA)<br>* The whole document * | 1-10 | |

TECHNICAL FIELDS SEARCHED (Int. Cl.5)

C 07 D 281/00

The present search report has been drawn up for all claims

| Place of search | Date of completion of search | Examiner |
|---|---|---|
| The Hague | 05 July 91 | ALLARD M.S. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention
E: earlier patent document, but published on, or after the filing date
D: document cited in the application
L: document cited for other reasons
&: member of the same patent family, corresponding document